## Europäisches Patentamt

## European Patent Office

## Office européen des brevets

(11) Veröffentlichungsnummer: **0 090 232**
A1

(12) # EUROPÄISCHE PATENTANMELDUNG

(21) Anmeldenummer: 83102453.4

(22) Anmeldetag: 12.03.83

(51) Int. Cl.³: **C 07 C 11/02,** C 07 C 1/20, C 07 C 5/03

(30) Priorität: 24.03.82 DE 3210756

(43) Veröffentlichungstag der Anmeldung: 05.10.83
Patentblatt 83/40

(84) Benannte Vertragsstaaten: **BE DE FR GB NL**

(71) Anmelder: **BASF Aktiengesellschaft,
Carl-Bosch-Strasse 38, D-6700 Ludwigshafen (DE)**

(72) Erfinder: **Hoelderich, Wolfgang, Dr., Mannheimer
Strasse 18 C, D-6710 Frankenthal (DE)**
Erfinder: **Eisenbeis, Ansgar, Dr.,
Albrecht-Duerer-Ring 14, D-6710 Frankenthal (DE)**
Erfinder: **Mross, Wolf Dieter, Dr.,
Anselm-Feuerbach-Strasse 21, D-6710 Frankenthal (DE)**
Erfinder: **Schwarzmann, Matthias, Dr.,
Carl-Bosch-Strasse 54, D-6703 Limburgerhof (DE)**

(54) Verfahren zur Herstellung von Olefinen aus Methanol und/oder Dimethylether.

(57)  Die Anmeldung betrifft ein mehrstufiges Verfahren zur kontinuierlichen Umsetzung von Methanol und/oder Dimethylether zu Olefinen in Gegenwart von Zeolithkatalysatoren vom Pentasil-Typ bei Temperaturen von 250 bis 550 °C, wobei man aus dem Umsetzungsprodukt der ersten Stufe $C_2$- bis $C_4$-Olefine zusammen mit Methan und Wasser abtrennt, die übrigen Anteile des Umsetzungsproduktes katalytisch hydriert und anschließend in Gegenwart von Wasserdampf weiter zu $C_2$- bis $C_4$-Olefinen spaltet. Die Umsetzung von Methanol und/oder Dimethylether wird zweckmäßig in Gegenwart von Borosilikatzeolithen und/oder Aluminiumsilikatzeolithen durchgeführt.

EP 0 090 232 A1

Verfahren zur Herstellung von Olefinen aus Methanol und/oder Dimethylether

Es sind eine Reihe Verfahren zur Herstellung von niederen Olefinen aus Methanol und/oder Dimethylether bekannt.

In neuerer Zeit gewinnen Bemühungen, Methanol zur Herstellung von Olefinen zu verwenden, zunehmendes Interesse. Methanol kann man aus Kohle, durch Kohlevergasung und Herstellung von Synthesegas durch Konvertierung, mit Hilfe bewährter Technik herstellen. Gelingt es, Methanol in technisch einfacher Weise in niedere Olefine umzuwandeln, können die heute üblichen Weiterverarbeitungsverfahren auch bei der Verwendung von Kohle als Rohstoff beibehalten werden. In den vergangenen Jahren sind daher eine Reihe von Verfahren entwickelt worden, die die Herstellung von Olefinen aus Methanol und/oder Dimethylether zum Gegenstand haben. Ein solches Verfahren ist beispielsweise in der DE-OS 26 15 150 beschrieben. Als Katalysator wird dabei der Aluminosilikatzeolith ZMS-5 verwendet. Alle bisher bekannt gewordenen Verfahren haben jedoch den Nachteil, daß sie nicht wirtschaftlich sind entweder des geringen Methanolumsatzes wegen, oder wegen des geringen Olefinanteils. Das Reaktionsprodukt enthält zumeist neben den gewünschten Olefinen hohe Anteile an flüssigen Kohlenwasserstoffen. Die Weiterverarbeitung dieser Kohlenwasserstoffe ist jedoch wegen des Anteils an leichtverkokenden Stoffen im Röhrenspaltofen schwierig und führt zu laufenden Verfahrensunterbrechungen.

Es wurde nun gefunden, daß man diese Nachteile bei der kontinuierlichen Umsetzung von Methanol und/oder Dimethylether zu Olefinen durch katalytische Umsetzung bei Temperaturen von 250 bis 550°C in Gegenwart von Zeolithkatalysatoren vom Pentasiltyp vermeidet, wenn man aus dem Umsetzungsprodukt der ersten Stufe die $C_2$- bis $C_4$-Olefine zusammen mit Methan und Wasser abtrennt, die übrigen Anteile des Umsetzungsproduktes katalytisch hydriert und anschließend in Gegenwart von Wasserdampf weiter zu $C_2$- bis $C_4$-Olefinen spaltet.

Für die erfindungsgemäße Durchführung des Verfahrens werden als Katalysatoren in der ersten Umsetzungsstufe zweckmäßig Borosilikatzeolithe und/oder Aluminosilikatzeolithe verwendet. Man arbeitet bei Temperaturen von 250 bis 550°C und vorteilhaft bei Temperaturen von 325 bis 500°C. Die Hydrierung kann man im allgemeinen in Gegenwart von Co- -Mo-Katalysatoren auf $Al_2O_3$-Träger bei Temperaturen von 40 bis 400°C, insbesondere 250 bis 350°C ausführen.

Gr/St

0090232

Bei Anwesenheit von mehrfach ungesättigten, nichtaromatischen Verbindungen im Umsetzungsprodukt der ersten Stufe kann man die Hydrierung zweistufig ausführen, wobei zuerst über Edelmetallkatalysatoren der 8. Gruppe des Periodensystems, wie Pd und/oder Pt auf $Al_2O_3$ und/oder $SiO_2$ als Träger die di- und polyolefinischen Bindungen und darauf erst die einfach ungesättigten Verbindungen in Gegenwart von Co-Mo-Katalysatoren hydriert werden. Bei Unterteilung der Hydrierung in zwei Stufen arbeitet man vorteilhaft in der ersten (Pd, Pt) Stufe bei 50 bis 150°C und in der zweiten Stufe bei 250 bis 350°C.

Ein besonderer Vorteil des Verfahrens besteht darin, daß in der Hydrierstufe die olefinischen und gegebenenfalls di- oder polyolefinischen Kohlenwasserstoffe des $C_5^+$-Gemisches aus der ersten Umsetzungsstufe hydriert werden und damit eine Verkokung oder Verharzung des Steamcrackers und Verfahrensunterbrechung verhindert wird. Durch diese Maßnahme erst ist es möglich, auf Kosten des $C_5^+$-Gemisches die Ausbeuten an den $C_2$-$C_4$-Olefinen in gewünschtem Maße zu erhöhen.

Es wurde weiterhin gefunden, daß man auch durch eine Erniedrigung der Umsetzungstemperatur der katalytischen Umsetzung des Methanols und/oder Dimethylethers in der ersten Reaktionsstufe auf Temperaturen von 350 bis 425°C den Aromatenanteil der $C_5^+$-Kohlenwasserstofffraktion zugunsten der Aliphate herabsetzen kann. Ein weiterer Vorteil dieser bevorzugten Ausführungsform des Verfahrens besteht darin, daß die von Temperatur und Belastung abhängigen Standzeiten der verwendeten Katalysatoren im Temperaturbereich von 350 bis 425°C bei gleicher Belastung ein Optimum aufweisen.

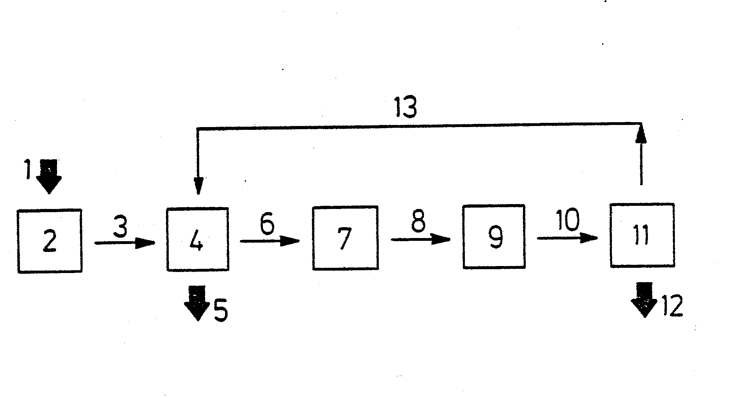

Das in der Zeichnung (Fig. 1) wiedergegebene Verfahrensschema gibt eine beispielsweise Anordnung für den grundlegenden Verfahrenslauf.

Aus Leitung (1) gelangen die Ausgangsstoffe Methanol und/ oder Dimethylether in den ersten Reaktor (2) des Dreistufenprozesses, in dem die Umwandlung von Methanol und/oder Dimethylether in ein Kohlenwasserstoffgemisch an einem Zeolithkatalysator durchgeführt wird. Die Reaktionsprodukte, die $C_2$-$C_4$-Olefine, $C_1$-$C_4$-Paraffine, flüssige Kohlenwasserstoffe $C_5^+$ mit einem hohen Anteil an nichtaromatischen Verbindungen und Wasser enthalten, gelangen durch Leitung (3) in die erste Aufarbeitungsstufe (4).

Als Katalysatoren für die erste Stufe eignen sich insbesondere Borosilikatzeolithe vom Pentasil-Typ. Die Reaktionsprodukte aus der ersten Stufe werden in (4) in bekannter Weise getrennt. Nach Abtrennung von Methan, $C_2$-

0090232

-$C_4$-Olefine und Wasser über Produktleitung (5) gelangen die $C_2$-$C_4$-Paraffine und die $C_5^+$-Kohlenwasserstoffe über Leitung (6) in den zweiten Reaktor (7), in dem die olefinischen Kohlenwasserstoffe an einem Hydrierkatalysator hydriert werden. Hierfür werden vorteilhaft Hydriermetalle der VI. Nebengruppe und VIII-Gruppe auf hitzebeständigen Trägermaterialien aus $Al_2O_3$ und/oder $SiO_2$ bevorzugt Co-Mo auf $Al_2O_3$ als Katalysatoren verwendet. Enthält das Kohlenwasserstoffgemisch aus der ersten Umsetzungsstufe Diene und weitere Polyolefine, so empfiehlt es sich, eine Selektivhydrierung vor der Olefinhydrierung dazwischenzuschalten. Als Katalysatoren für diese Selektivhydrierung eignen sich Edelmetalle der VIII-Gruppe z.B. Pd und Pt auf $Al_2O_3$ in Gewichtsmengen von 0,1 bis 1 %.

Die im zweiten Reaktor (7) erhaltenen Kohlenwasserstoffe gelangen durch Leitung (8) in einen Röhrenspaltofen (9), in dem sie zum großen Teil in bekannter Weise zu Ethylen und Propylen umgesetzt werden. Die Reaktionsprodukte aus dem Röhrenspaltofen (9) werden in eine zweite Aufarbeitungsstufe (11) gegeben. In (11) werden die Aromaten und Rückstandsöl aus dem Reaktionsgemisch abgetrennt und über Leitung (12) abgezogen. Die anderen Reaktionsprodukte z.B. Ethylen, Propylen und Pyrolysebenzin gelangen über Leitung 13 in die erste Aufarbeitungsstufe 4 und werden dort weiterverarbeitet.

Auch kann dem Röhrenspaltofen (9) eine BTX-Aromatenextraktion vorgeschaltet werden, in der die BTX-Aromaten abgetrennt werden und die restlichen Kohlenwasserstoffe aus der Hydrierstufe 7 dem Steamreaktor (9) zugeführt werden.

Die Methanolumwandlung in der ersten Reaktionsstufe (2) erfolgt zwischen 250 und 550°C, bevorzugt 350 bis 425°C, bei Drucken zwischen 0 und 42 bar, bevorzugt 1 bis 3 bar, und Belastungen WHSV (kg Methanol/kg Katalysator und Stunde) zwischen 0,1 und 45 $h^{-1}$, bevorzugt zwischen 1 und 8 $h^{-1}$.

Als Einsatzstoff wird zweckmäßig wasserverdünntes Methanol, bevorzugt Rohmethanol (17 % Wasser) verwendet. Die Reaktion wird an kristallinen Boro- und/oder Aluminosilikatzeolithen mit $SiO_2/B_2O_3$ bzw. $Al_2O_3 \geqslant 10$ (H-Form des Zeolithen) ausgeführt.

In der gegebenenfalls angewendeten zweistufigen Vollhydrierung werden die Reaktionsprodukte nach der ersten Aufarbeitungsstufe (4) zunächst bei Temperaturen zwischen 40 und 250°C, bei Drucken zwischen 10 und 50 bar, einer Belastung 0,5 bis 5 kg Kohlenwasserstoffe (KW) pro Liter Katalysator und Stunde und einem Wasserstoff zu KW von 0,01 bis 2 m3N/kg umge-

setzt, danach bei Temperaturen von 180 bis 400°C, Drucken von 20 bis 100 bar, Belastung 0,5 bis 5 kg KW pro 1 Katalysator und Stunde und einem Wasserstoff-KW-Verhältnis von 0,01 bis 5 $m^3N$/kg.

Die erste Hydrierung erfolgt zweckmäßig an einem Edelmetall-Katalysator, der sich z.B. aus 0,1 bis 1 Gew.% eines Edelmetalles der VIII-Gruppe, wie Pd und Pt auf Aluminiumoxid zusammensetzt. Für die zweite Hydrierung kann man einen Katalysator bestehend aus Hydriermetallen der VI-Nebengruppe und VIII-Gruppe auf hitzebeständigen Trägermaterialien aus Aluminiumoxid und/oder Siliciumoxid, vorzugsweise Co-Mo auf $Al_2O_3$ anwenden.

Die Umsetzung der Reaktionsprodukte aus der Hydrierstufe im Steamcracker wird nach bekannter Technologie bei 850°C und einem Dampf/KW-Verhältnis von 0,5 durchgeführt.

Die Durchführung des erfindungsgemäßen Verfahrens wird durch die nachfolgenden Beispiele näher erläutert.

Beispiel

Es wurden Zeolithe des Pentasil-Typs aus $SiO_2$ und $Al_2O_3$ bzw. $B_2O_3$ unter hydrothermalen Bedingungen bei 150 bis 170°C in einer 50 %igen Aminlösung hergestellt. Durch Abbrennen der Aminkomponente bei 540°C/24 h wird der Zeolith in die katalytisch aktive H-Form gebracht.

Der calcinierte Zeolith wird mit Böhmit im Verhältnis 60 : 40 verstrangt.

Katalysator A: $SiO_2/Al_2O_3$ = 34, hergestellt in Hexamethylen-
          diaminlösung

Katalysator B: $SiO_2/B_2O_3$ = 23, hergestellt in Diamino-
          propanlösung

Diese Katalysatoren werden für die Methanolumwandlung in Kohlenwasserstoffe eingesetzt. Die Testbedingungen und die Zusammensetzung der in der ersten Stufe erhaltenen Reaktionsprodukte sind in Tabelle 1 zusammengestellt. Der Methanolumsatz ist quantitativ. Die Gewichtsprozente sind auf eingesetztes $CH_2$ bezogen.

Ethylen, Propylen, Butene, Methan und Wasser werden von den übrigen Katalysatoren B erhaltenen Reaktionsprodukten abgetrennt. Die verbleibenden Kohlenwasserstoffe werden bei 340°C, 52 bar Gesamtdruck und bei einer Beladung von 1,3 kg KW pro 1 Katalysator an einem Kobalt-Molybdänkatalysator umgesetzt. Der Katalysator setzt sich zusammen aus 5 Gew.% CoO

und 13,5 Gew.% $MoO_3$ auf hitzebeständigem Aluminiumoxid. Die 25,3 Gew.% $C_5^+$-Olefine werden quantitativ zu Paraffinen hydriert.

Die nach der Hydrierung erhaltenen Kohlenwasserstoffe werden bei 850°C einer Dampfspaltung unterworfen. Der Gesamtethylenwert steigt durch die Dampfspaltung von 4,3 Gew.% auf 22,5 Gew.%, der Propylenwert von 13,2 Gew.% auf 21,7 Gew.% und die Butenausbeuten von 10,5 auf 13,2 %.

<u>Tabelle</u>

| Beispiel | 2 | 3 | 4 |
|---|---|---|---|
| Katalysator | A | A | A |
| Methanol % | 50 | 50 | 83 |
| Temperatur | 350°C | 425°C | 400°C |
| WHSV | 0,65 h$^{-1}$ | 0,46 h$^{-1}$ | 5,9 h$^{-1}$ |
| $CH_4$ Gew.% | 0,4 | 1,9 | 0,7 |
| $C_2H_4$ Gew.% | 10,8 | 7,9 | 4,3 |
| $C_2H_6$ Gew.% | 0,1 | 0,2 | 0,1 |
| $C_3H_6$ Gew.% | 7,3 | 21,2 | 13,2 |
| $C_3H_8$ Gew.% | 4,1 | 3,0 | 2,0 |
| $C_4H_8$ Gew.% | 6,5 | 16,4 | 10,5 |
| $C_4H_{10}$ Gew.% | 11,6 | 5,5 | 8,8 |
| $C_5^+$-Olefine Gew.% | 22,5 | 15,2 | 25,3 |
| $C_5^+$-Paraffine Gew.% | 20,4 | 11,4 | 22,1 |
| Aromaten Gew.% | 13,9 | 14,3 | 9,7 |
| $CO_2$, Co, $H_2$, Koks Gew.% | Rest | Rest | Rest |

## Patentansprüche

1. Mehrstufiges Verfahren zur kontinuierlichen Umsetzung von Methanol und/oder Dimethylether zu Olefinen in Gegenwart von Zeolithkatalysatoren vom Pentasil-Typ bei Temperaturen von 250 bis 550°C, dadurch gekennzeichnet, daß man aus dem Umsetzungsprodukt der ersten Stufe $C_2$ bis $C_4$-Olefine zusammen mit Methan und Wasser abtrennt, die übrigen Anteile des Umsetzungsproduktes katalytisch hydriert und anschließend in Gegenwart von Wasserdampf weiter zu $C_2$- bis $C_4$-Olefinen spaltet.

2. Verfahren nach Anspruch 1, dadurch gekennzeichnet, daß man die Umsetzung von Methanol und/oder Dimethylether in Gegenwart von Borosilikatzeolithen und/oder Aluminiumsilikatzeolithen durchführt.

3. Verfahren nach Anspruch 1 und 2, dadurch gekennzeichnet, daß man die Hydrierung in Gegenwart von Co-Mo-Katalysatoren auf $Al_2O_3$-Träger ausführt.

4. Verfahren nach Anspruch 1 bis 3, dadurch gekennzeichnet, daß man bei Anwesenheit von mehrfach ungesättigten, nichtaromatischen Verbindungen im Umsetzungsprodukt der ersten Stufe die Hydrierung zweistufig ausgeführt wird, wobei zuerst über Edelmetallkatalysatoren der 8. Gruppe des Periodensystems die di- und polyolefinischen Bindungen und darauf die einfach ungesättigten Verbindungen in Gegenwart von Co-Mo-Katalysatoren hydriert werden.

Zeichn.

0090232

1/1

**Europäisches Patentamt**

**EUROPÄISCHER RECHERCHENBERICHT**

0090232

Nummer der Anmeldung

EP 83 10 2453

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | NL-C- 67 093 (DE BATAAFSCHE PETROLEUM MAATSCHAPPIJ) * Beispiel III * | 3,4 | C 07 C 11/02 C 07 C 1/20 C 07 C 5/03 |
| | --- | | |
| A | EP-A-0 041 621 (BASF) | | |
| | --- | | |
| A | DE-A-1 568 542 (BAYER) | | |
| | --- | | |
| A,P | EP-A-0 051 741 (BASF) | | |
| | ----- | | |

| RECHERCHIERTE SACHGEBIETE (Int. Cl. ³) |
|---|
| C 07 C 1/00 C 07 C 5/00 |

Der vorliegende Recherchenbericht wurde für alle Patentansprüche erstellt.

| Recherchenort DEN HAAG | Abschlußdatum der Recherche 03-06-1983 | Prüfer VAN GEYT J.J.A. |
|---|---|---|